# EUROPEAN PATENT APPLICATION

(11) **EP 2 586 776 A1**
(43) Date of publication of application: **01.05.2013**
(21) Application number: 10853755.6
(22) Date of filing: 24.06.2010
(51) Int. Cl.: C07D 311/02, A61K 31/352, A61P 3/00, A61P 41/00

(54) **SOFT CATIONIC MITOCHONDRIAL UNCOUPLERS**

(71) Applicant: Limited Liability Company Mitotech, Moscow 119992 (RU)
(72) Inventor: SKULACHEV, Vladimir Petrovich, Moscow 119234 (RU); SKULACHEV, Maxim Vladimirovich, Moscow 119234 (RU); ZINOVKIN, Roman Alexeevich, Moscow 119526 (RU); SEVERIN, Fedor Fedorovich, Moscow 119234 (RU); ANTONENKO, Yury Nikolaevich, Moscow 119313 (RU); ZOROV, Dmitry Borisovich, Moscow 117292 (RU); PLOTNIKOV, Egor Yurievich, 142452 Noginsky raion Moskovskaya obl. (RU); ISAEV, Nikolay Konstantinovich, 140250 Voskresensky raion Moskovskaya obl. (RU); SILACHEV, Denis Nikolaevich, Moscow 109341 (RU); KNORRE, Dmitry Alexeevich, Moscow 117313 (RU)
(74) Representative: Richards, John
(86) International application number: PCT/RU2010/000353
(87) International publication number: WO 2011/162633

(57) **Abstract**

The present invention relates to biology and medicine and in particular can be used in medicine for the preparation of a pharmaceutical composition for the specific, self-regulating uncoupling of mitochondria. The invention can be useful in the treatment of diseases and conditions associated with the disruption of cellular metabolism, in the treatment of obesity, including pathological forms thereof, and also for the treatment of diseases associated with the increased formation of free radicals and reactive oxygen species.

## Description

### Field of the invention

The present invention relates to pharmacology, biology and medicine and refers to manufacturing and application of a class of compounds which can be used in pharmaceutical compositions of medicines (preparations) for prophylaxis and treatment of various diseases and pathological conditions in humans and animals accompanied by an increased level of synthesis of adenosine triphosphate in mitochondria and endogenous oxidative stress.

### Prior art

Most eukaryotic cells contain specialized organelles - mitochondria. Mitochondria are often called the cell's power plants because their main function is the synthesis of adenosine triphosphate (ATP), the universal energy carrier molecule, which helps in almost all cellular functions. Biosynthesis of ATP occurs through oxidative phosphorylation. The essence of this process is to transfer electrons from NADH molecule to molecular oxygen along the mitochondrial electron transport chain, resulting in the formation of water molecule and creation of an electrochemical proton gradient, the latter in used by the ATP synthase enzyme to synthesize ATP. In homoiothermal animals, part of the energy stored in the form of the electrochemical proton gradient is used to maintain a constant body temperature and is not coupled to the synthesis of ATP. Uncoupling of oxidative phosphorylation from the ATP synthesis occurs at the expense of free fatty acids located in the cell cytoplasm. At the outer surface of mitochondrial membrane, a fatty acid anion (RCOO⁻) binds an H⁺ ion pumped from mitochondria by the respiratory chain enzymes. The resulting neutral protonated form of the fatty acid (RCOOH) crosses the membrane and dissociates at its inner surface yielding RCOO⁻ and H⁺. The latter compensates for the lack of H⁺ ions inside mitochondria removed therefrom by the respiratory chain. The resulting RCOO⁻ anion returns to the outside by means of mitochondrial proteins - anion transporters, in particular, the ATP/ADP-antiporter or special uncoupling proteins called UCP (uncoupling protein). Normally, 20-25% of the total metabolism of warm-blooded animals is directed at maintaining a constant body temperature, and the mechanism of heat production is associated with the uncoupling of respiration from ATP synthesis at the expense of an increase in the proton conductivity. The increase in proton conductivity or "mild uncoupling" can serve both for thermogenesis and for body weight control due to stimulation of carbohydrate and lipid metabolism.

Natural mechanisms of uncoupling do not always cope with their functions. Many pathological conditions, at which mitochondrial hyperpolarization accompanied by elevated level of reactive oxygen species (ROS) in mitochondria occurs that leads to deterioration in functioning of the cell as a whole, are described. This situation occurs with obesity, impaired carbohydrate metabolism, and also with pathologies associated with the development of oxidative stress: ischemic tissue damage, Parkinson's disease, Alzheimer's disease, autoimmune diseases.

The use of uncouplers capable of adjustable increase in mitochondrial proton conductivity, can eliminate the negative effects caused by said pathological conditions, in particular, by controlling the level of ROS generation by mitochondria (Korshunov SS, Skulachev VP, Starkov AA, High protonic potential actuates a mechanism of production of reactive oxygen species in mitochondria. FEBS Lett 1997; 416; 15-18.).

The clinical effect of anionic uncoupler, 2,4-dinitrophenol (DNP) has been shown in obese people. (Parascandola J, Dinitrophenol and bioenergetics: a historical perspective. Mol Cell Biochem 1974; 5: 69-77*.).* By 1934, more than one hundred thousand patients were treated with DNP. After the treatment, virtually all of the patients lost weight with varying degrees of efficiency, however many of them showed severe side effects. A small overdose caused muscle weakness, high fever in a patient, that in some cases led to death. In addition, in many patients, prolonged administration of the preparation led to the development of cataract and vision loss. In 1938, all of these facts led to the strict ban on the use of this preparation for the treatment of any disease. However, the fact that the treatment with DNP actually led to weight loss in patients indicates the fidelity of the approach.

Thus, development of nontoxic preparations that enhance proton conductivity of the mitochondrial membrane is a promising avenue for creating a new class of drugs and compositions. To avoid a situation when protonophores have toxic effects and are active when the cell requires the synthesis of ATP, it is necessary that their activity would be dependent on the functional state of mitochondria, for instance, on the potential across the inner membrane. In the state of hyperpolarization, a protonophore should discharge only the excess potential but should not reduce it overly that would inevitably lead to the inhibition of respiration. Ideal protonophore should not inhibit mitochondrial respiration even at relatively high concentrations. Earlier attempts to synthesize substances that would have properties of "mild" mitochondrial uncouplers were undertaken, but they failed (Blaikie FH, Brown SE, Samuelsson LM, Brand MD, Smith RA, Murphy MP, Targeting dinitrophenol to mitochondria: limitations to the development of a self-limiting mitochondrial protonophore. Biosci Rep. 2006; 26; 231-43*.).*

The vast majority of protonophores including DNP are negatively charged molecules, anions, and their mechanism of uncoupling is analogous to that of fatty acid anions (RCOO⁻). Protonation of anionic protonophore results in a neutral compound which penetrates into a mitochondrion regardless of mitochondrial potential, whereas "mild uncoupler" should preferably penetrate only into a hyperpolarized mitochondrion.

All of the above hold great promise for the use of the compounds causing mild uncoupling of mitochondria. However, until now it has not been possible to obtain compounds with said properties.

### Definitions:

Prior to the detailed description of the invention follows, one should understand that the invention is not limited to the particular methodology, protocols, and reagents described here, as they are subject to change. In addition, it should be understood that in the present invention, the terminology is used to describe particular embodiments only and does not limit the scope of the present invention which will be limited only by the appended claims. Unless otherwise specified, all technical and scientific terms used here have the same meanings that are understandable to those skilled in the art.

**Protonophore** is a compound capable of electrogenic transport of proton across the inner mitochondrial membrane that leads to the fall in membrane potential and uncoupling of oxidation from phosphorylation.

**Uncoupler** is a compound that causes uncoupling of respiration from oxidative phosphorylation in mitochondria.

The mechanism of uncoupling is associated with a decrease in mitochondrial membrane potential due to protonophore activity of compounds or induction of endogenous proton conductivity of membranes.

**Self-regulating uncoupler** is a compound whose uncoupling ability depends on the value of mitochondrial membrane potential.

**Mild uncoupling or moderate uncoupling** is a reversible decrease in mitochondrial membrane potential that does not cause the suppression of respiration, respiratory chain component damage or violation of physicochemical properties of mitochondrial membranes.

**Mitochondria-targeted protonophore** is a compound that targetedly accumulates in mitochondria and increases proton conductivity of the inner mitochondrial membrane (i.e., decreases proton electrochemical potential across this membrane).

Throughout the text of a description of the invention various documents are cited. Each document cited here (including all patents, patent applications, scientific publications, specifications and manufacturer's instructions etc.), above or below, is introduced in full in this invention by reference.

**definitions of other terms:** substituted or unsubstituted alkyl group, substituted or unsubstituted aryl group, substituted or unsubstituted heteroaryl group, substituted or unsubstituted cycloalkyl group, substituted or unsubstituted heterocyclic group, saturated or partially unsaturated polycyclic system. These terms of each use case in the rest of the description may have a corresponding defined meaning and preferred meanings. Nevertheless, in some cases of their use throughout the description of the invention, preferred meanings of these terms are given.

The term "substituted or unsubstituted alkyl group" refers to substituted or unsubstituted saturated or unsaturated linear, branched or cyclic carbon chain. Preferably, this chain contains from 1 to 14 carbon atoms, i.e., 1, 2, 3, 4, 5, 6, 7, 8, 9, 10, 11, 12, 13 or 14, for example, methyl, ethyl methyl, ethyl, propyl, iso-propyl, butyl, iso-butyl, tert-butyl, pentyl, hexyl, heptyl, octyl, nonyl, decyl, dodecyl, octadecyl. In addition, the term "substituted or unsubstituted alkyl group" in the present invention also includes heteroalkyl group which is saturated or unsaturated linear or branched carbon chain. Preferably, this chain contains from 1 to 14 carbon atoms, i.e., 1, 2, 3, 4, 5, 6, 7, 8, 9, 10, 11, 12, 13 or 14, for example, methyl, ethyl methyl, ethyl, propyl, iso-propyl, butyl, iso-butyl, tert-butyl, pentyl, hexyl, heptyl, octyl, nonyl, decyl, dodecyl, octadecyl, which is interrupted once or several times, e.g., 1, 2, 3, 4, 5 times, by same or different heteroatoms. Preferably, heteroatoms are selected from O, S, and N, for example, CH₂-O-CH₃, CH₂-O-C₂H₅, **C₂H₄-O-CH₃, C₂H₄-O-C₂H₅** etc.

The term "substituted or unsubstituted aryl group" preferably refers to aromatic monocyclic ring containing 6 carbon atoms, aromatic bicyclic ringed system containing 10 carbon atoms or aromatic ringed tricyclic system containing 14 carbon atoms. Examples are phenyl, naphthalenyl or anthracenyl. In addition, the term "substituted or unsubstituted aryl group" in the present invention also includes substituted or unsubstituted aralkyl group. The term "substituted or unsubstituted aralkyl group" refers to alkyl moiety which is substituted by aryl, and alkyl and aryl are as defined above. An example is benzene radical. Preferably, in this context, alkyl chain contains from 1 to 8 carbon atoms, i.e., 1, 2, 3, 4, 5, 6, 7 or 8, for example, methyl, ethyl, propyl, iso-propyl, butyl, iso-butyl, sec-butenyl, tert-butyl, pentyl, hexyl, heptyl, octyl. Aralkyl group is optionally substituted at alkyl and/or aryl moiety. Preferably, aryl attached to alkyl group is phenyl, naphthalenyl or anthracenyl.

The terms "substituted or unsubstituted heterocyclic group", alone or in combination with other terms, represent, unless otherwise stated, cyclic versions of "alkyl" and "heteroalkyl" groups, respectively, preferably with 3, 4, 5, 6, 7, 8, 9 or 10 atoms forming ring, for example, cyclopropyl, cyclobutyl, cyclopentyl, cyclohexyl, cycloheptyl, cyclooctyl etc. It is implied that the terms "cycloalkyl" and "heterocycloalkyl" also include their bicyclic, tricyclic and polycyclic versions. If bicyclic, tricyclic or polycyclic rings are formed, it is preferable that corresponding rings are linked together by two adjacent carbon atoms, but as an alternative, two rings may be linked via the same carbon atom, i.e., they form spiro ring system or they form "bridged" ring systems. The term "substituted or unsubstituted heterocyclic group" preferably refers to saturated ring having five atoms, of which at least one is N, O or S atom, and which optionally contains one additional O atom or one additional N atom; saturated ring having six atoms, of which at least one is N, O or S atom and which optionally contains one additional O atom or one additional N atom or two additional N atoms; or saturated bicyclic ring having nine or ten atoms, of which at least one is N, O or S atom, and which optionally contains one, two or three additional N atoms. "Cycloalkyl" and "heterocycloalkyl" groups are optionally substituted. Additionally for heterocycloalkyl, heteroatom may be in a position at which heterocycle is linked to the rest of the molecule. Preferred examples of cycloalkyl include cyclopropyl, cyclobutyl, cyclopentyl, cyclohexyl, 1-cyclohexenyl, 3-cyclohexenyl, cycloheptyl, spiro [3,3] heptyl, spiro [3,4] octyl, spiro [4,3] octyl, spiro [3,5] nonyl, spiro [5,3] nonyl, spiro [3,6] decyl, spiro [6,3] decyl, spiro [4,5] decyl, spiro [5,4] decyl, bicyclo [2.2.1] heptyl, bicyclo [2.2.2] octyl, adamantyl and the like. Examples of heterocycloalkyl include 1-(1,2,5,6- tetrahydropyridyl), 1-piperidinyl, 2-piperidinyl, 3-piperidinyl, 4-morpholinyl, 3-morpholinyl, 1,8-diazaspiro-[4,5] decyl, 1,7-diazaspiro [4,5] decyl, 1,6-diazaspiro [4,5] decyl, 2,8-diazaspiro [4,5] decyl, 2,7-diazaspiro [4,5] decyl, 2,6-diazaspiro [4,5] decyl, 1,8-diazaspiro [5,4] decyl, 1,7-diazaspiro [5,4] decyl, 2,8-diazaspiro-[5,4] decyl, 2,7-diazaspiro [5,4] decyl, 3,8-diazaspiro [5,4] decyl, 3,7-diazaspiro [5,4] decyl, 1-aza-7,11-dioxo-spiro [5,5] undecyl, 1,4-diazabicyclo [2.2.2] oct-2-yl, tetrahydrofuran-2-yl, tetrahydrofuran-3-yl, tetrahydrothien-2-yl, tetrahydrothien-3-yl , 1-piperazinyl, 2-piperazinyl, and the like.

The term "polycyclic system" refers to bicyclic, tricyclic or polycyclic cycloalkyl or heterocycloalkyl variant comprising at least one double and/or triple bond. Nevertheless, alicyclic system is not aromatic or heteroaromatic, i.e., there is no system of conjugated double bonds or free electron pairs. Thus, the maximum number of double and/or triple bonds in alicyclic system is determined by the number of atoms in ring, for example, in cyclic system having up to 5 atoms, alicyclic system contains one double bond, in cyclic system having 6 atoms, alicyclic system contains up to two double bonds. Thus, defined below term "cycloalkenyl" is a preferred embodiment of alicyclic ring system. Alicyclic systems are optionally substituted.

The term "substituted or unsubstituted heteroaryl group" preferably refers to five- or six-membered aromatic monocyclic ring, wherein at least one of carbon atoms is replaced by 1, 2 or 3 (for five-membered ring) or 1, 2, 3, or 4 (for six-membered ring) same or different heteroatoms, preferably selected from O, N and S; aromatic bicyclic ring system in which 1, 2, 3, 4, 5 or 6 carbon atoms of 8, 9, 10, 11 or 12 carbon atoms are replaced by identical or different heteroatoms, preferably selected from O, N and S; or tricyclic aromatic ring system in which 1, 2, 3, 4, 5 or 6 carbon atoms of 13, 14, 15 or 16 carbon atoms are replaced by same or different heteroatoms, preferably selected from O, N and S. Examples include furanyl, thiophenyl, oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indolyl, isoindolyl, benzothiophenyl, 2-benzothiophenyl, 1H-indazolyl, benzimidazolyl, benzoxazolyl, indoxazinyl, 2,1-benzisoxazolyl, benzothiazolyl, 1,2-benzisothiazolyl, 2,1-benzisothiazolyl, benzotriazolyl, quinolinyl, isoquinolinyl, 2,3-benzodiazinyl, quinoxalinyl, quinazolinyl, 1,2,3-benzotriazinyl, or 1,2,4-benzotriazinyl. In addition, the term "substituted or unsubstituted heteroaryl group" in the present invention also includes substituted or unsubstituted heteroaralkyl group. The term "heteroaralkyl" refers to alkyl moiety which is substituted by heteroaryl wherein the terms alkyl and heteroaryl are as defined above. An example is (2-pyridinyl)ethyl, (3-pyridinyl)ethyl or (2-pyridinyl)methyl. Preferable in this context alkyl chain contains from 1 to 8 carbon atoms, i.e., 1, 2, 3, 4, 5, 6, 7 or 8, for example, methyl, ethylmethyl, ethyl, propyl, iso-propyl, butyl, iso-butyl, sec-butenyl, tert-butyl, pentyl, hexyl, heptyl, octyl. Heteroaralkyl group is optionally substituted in alkyl and/or heteroaryl part of a group. Preferably heteroaryl attached to alkyl moiety is oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indolyl, isoindolyl, benzothiophenyl, 2-benzothiophenyl, 1H-indazolyl, benzimidazolyl, benzoxazolyl, indoxazinyl, 2,1-benzisoxazolyl, benzothiazolyl, 1,2-benzisothiazolyl, 2,1-benzisothiazolyl, benzotriazolyl, 2,3-benzodiazinyl, quinolinyl, isoquinolinyl, quinoxalinyl, quinazolinyl, 1,2,3-benzotriazinyl, or 1,2,4-benzotriazinyl.

Embodiments include radicals: alkoxy, cycloalkoxy, aryloxy, aralkoxy, alkenyloxy, cycloalkenyloxy, alkynyloxy, alkylthio, cycloalkylthio, arylthio, aralkylthio, alkenylthio, cycloalkenylthio, alkynylthio, alkylamino, cycloalkylamino, arylamino, aralkylamino, alkenylamino, cycloalkenylamino, alkynylamino.

Other embodiments include radicals: hydroxyalkyl, hydroxycycloalkyl, hydroxyaryl, hydroxyaralkyl, hydroxyalkenyl, hydroxycycloalkenyl, hydroxyalkynyl, mercaptoalkyl, mercaptocycloalkyl, mercaptoaryl, mercaptoaralkyl, mercaptoalkenyl, mercaptocycloalkenyl, mercaptoalkynyl, aminoalkyl, aminocycloalkyl, aminoaryl, aminoaralkyl, aminoalkenyl, aminocycloalkenyl, aminoalkynyl.

In another embodiment, hydrogen atoms in alkyl, cycloalkyl, aryl, aralkyl, alkenyl, cycloalkenyl, alkynyl radicals may be substituted independently of each other by one or more halogen atoms. One of the radicals is trifluoromethyl radical.

If two or more radicals may be selected independently of each other, the term "independently" implies that these radicals may be identical or may be different.

The term "pharmaceutically acceptable salt" refers to salt of compound of the present invention. Suitable pharmaceutically acceptable salts of compound of the present invention include acid addition salts which may be obtained for example by mixing solution of compound of the present invention with solution of pharmaceutically acceptable acid, such as hydrochloric acid, sulfuric acid, fumaric acid, maleic acid, succinic acid, acetic acid, benzoic acid, citric acid, tartaric acid, carbonic acid or phosphoric acid. In addition, when according to the invention compounds have acid moiety, their suitable pharmaceutically acceptable salts may include alkali metal salts (e.g., sodium or potassium salts); salts of alkaline earth metals (e.g., calcium or magnesium salts); and salts formed with suitable organic ligands (e.g., ammonium, quaternary ammonium and amine cations formed using counterions, such as halide, hydroxide, carboxylate, sulfate, phosphate, nitrate, alkyl sulfonate and aryl sulfonate). Illustrative examples of pharmaceutically acceptable salts include (but are not limited to): acetate, adipate, alginate, ascorbate, aspartate, benzenesulfonate, benzoate, bicarbonate, bisulfate, bitartrate, borate, bromide, butyrate, calcium salt of ethylenediaminetetraacetic acid (edetate), camphorate, camphorsulfonate, camsilate, carbonate, chloride, citrate, clavulanate, cyclopentane propionate, digluconate, dihydrochloride, dodecylsulfate, edetate, edisilate, estolate, esilate, ethanesulfonate, formiate, fumarate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphtoate, iodide, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, subacetate, succinate, tannate, tartrate, theoclate, tosylate, triethiodide, undecanoate, valerate and the like (e.g., see an article of Berge S.M. et al., "Pharmaceutical salts", Journal of Pharmaceutical Science, 1977, v.66, p.1-19). Some specific compounds of the present invention contain basic and acidic functional groups that allows to convert these compounds into either basic or acid addition salts.

Neutral forms of compounds may be recovered by contacting salt with base or acid, with release of original compound by traditional way. Original form of compound differs from various salt forms in certain physical properties, such as solubility in polar solvents, but in other aspects, for purposes of the present invention, these salts are equivalent to original form of compound.

In addition to salt forms, the present invention provides compounds which are prodrug forms. Prodrug forms of said compounds are such compounds that under physiological conditions readily undergo chemical changes to form compound of structure (I). Prodrug is pharmacologically active or inactive compound that is chemically modified by physiological action *in vivo,* such as hydrolysis, metabolism, and the like, to form compound of the present invention, after administration of prodrug in patient. Additionally, prodrugs can be converted into compounds of the present invention by means of chemical or biochemical methods *in vitro.* For example, prodrugs can be slowly converted into compounds of the present invention under the influence of respective enzyme placed in reservoir transdermal patch. Suitability of method and technique used in obtaining and using prodrugs is well known to those skilled in the art. General consideration of prodrugs which comprise esters is given in a review of Svensson and Tunek, Drug Metabolism Reviews 16.5 (1988) and in a book of Bundgaard, Design of Prodrugs, Elsevier (1985).

Compounds, as well as raw materials for their production according to the invention can be synthesized using traditional methods and techniques known to those skilled in the art, i.e., as described in the literature (for example, in traditional works, such as Houben-Weyl, Methoden der organischen Chemie [Methods of Organic Chemistry], Georg-Thieme-Verlag, Stuttgart), under reaction conditions which are known to those skilled in the art and which are suitable for these reactions. In addition, in the present invention, any other known variants, which are not mentioned here in more detail, may be used.

Some compounds of the present invention may exist in unsolvated forms as well as in solvated forms including hydrated forms. In general, solvated forms are equivalent to unsolvated forms and it is assumed that they are within the scope of the present invention. Some compounds of the present invention may be in various crystalline or amorphous forms. Usually, all physical forms are equivalent for intended use in the present invention, and it is assumed that they are within the scope of the present invention.

Some compounds of the present invention have asymmetric carbon atoms (optical centers) or double bonds. It is assumed that all racemates, enantiomers, diastereomers, geometric isomers and individual isomers are within the scope of the present invention. Hence, compounds of the invention comprise mixtures of stereoisomers, especially mixtures of enantiomers, as well as purified stereoisomers, especially purified enantiomers or stereoisomerically-enriched mixtures, especially enantiomerically-enriched mixtures. In addition, the scope of the invention includes individual isomers of compounds shown below by formulas (1) to (5), as well as any of their fully or partially balanced mixtures. In addition, the present invention comprises individual isomers of compounds shown below by formulas, as mixtures with isomers thereof in which one or more chiral centers are inverted. In addition, it should be understood that all tautomers and mixtures of tautomers of compounds of formulas (1) - (5), are within the scope of compounds of formulas (1) - (5), and preferably match formula and partial formula which meet this goal. Obtained racemates can be split into isomers by essentially known mechanical or chemical ways. Diastereomers are preferably formed from racemic mixture by means of intereaction with optically active splitting agent. The splitting of enantiomer with the use of a column filled with optically active splitting agent is also advantageous.

In addition, the splitting of diastereomer can be accomplished using standard purification methods, for instance, such as chromatography or fractional crystallization.

Compounds of the present invention may also have unnatural ratios of atomic isotopes for one or more atoms which are part of such compounds. For example, these compounds can be labeled with radioactive isotopes, for example, such as tritium (³H), iodine-125 (¹²⁵I) or carbon-14 (¹⁴C). It is assumed that all isotopic variants of compounds of the present invention (radioactive or not radioactive) are within the scope of protection of the present invention.

### Description of the invention

A number of compounds described by general structure (I) capable of mild uncoupling of mitochondria are developed. The invention is based on a principle of self-regulating targeted delivery of biologically active compounds in mitochondria of a living cell at the expense of energy of electrochemical potential. Proposed mechanism of self-regulation is following: compounds of structure (I) in cytoplasm of a cell interact with a proton and become positively charged. Due to this charge, and the lipophilic nature of these compounds, they are delivered to negatively charged mitochondria in which they accumulate according to the Nernst equation. It is important to note that the functional state of a mitochondrion (i.e., its potential) will determine concentration of compounds of structure (I). Said property of compounds determines their mild uncoupling activity that provides effective uncoupling of only mitochondria with high membrane potential. Being penetrated the mitochondrial inner membrane, said compounds increase its proton conductivity that stimulates the fall in membrane potential of mitochondria. In the mitochondrial matrix, said compounds are deprotonated and the neutral form of uncoupler diffuses from a mitochondrion back to the cytoplasm.

Fortuitously, when conducting experiments with compounds of structure (I), it was found that they possess moderate uncoupling activity (see Experimental examples). The results of our experiments indicate that the observed uncoupling process depends on the mitochondrial potential and does not depend on the presence of fatty acid anions (RCOO⁻) - potential participants of the uncoupling process, localized at the outer surface of mitochondria.

Compounds of structure (I) are similar to other classes of cationic uncouplers of compounds. However, cationic uncouplers known to date cause a nonspecific increase in mitochondrial membrane permeability that leads to mitochondrial swelling, disruption of the functioning of a cell and an organism as a whole (Shinohara Y, Bandou S, Kora S, Kitamura S, Inazumi S, Terada H. (1998), FEBS Lett. 428, 89-92.). Unexpected and fundamental difference of the claimed structure is that its mild uncoupling effect is dependent on the mitochondrial electrochemical potential and significantly predominates over detergent effects or other unwanted side effects.

General formula of compounds (I) is as follows: 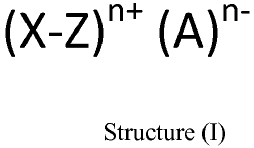 and/or reduced form thereof,
where
«X» - group comprising substituted or unsubstituted hydrocarbon chain with a length of up to 20 carbon atoms, optionally containing double or triple bonds, heteroatoms such as -S-, -N-, - O-, or optionally containing -C(O)O-, -C(O)- and/or -C(O)N(H)- groups;
«Z» - group that can be protonated under physiological pH values and able to provide targeted delivery of the whole compound into mitochondria.
«A» - counterion
«n» - integer from 1 to 3,
and/or tautomeric forms, solvates, salts, isomers or prodrug forms thereof, and pharmaceutically acceptable carrier.

With proviso that combination of X and Z in one compound should:
a) have an ability to be protonated at pH values typical to cell cytoplasm and have pK from 4 to 11;
b) have enough lipophilicity to penetrate mitochondria in the protonated form;
c) provide electrochemical potential-dependent penetration in mitochondria;
d) have an ability to be deprotonated under alkalescent pH conditions typical for mitochondrial matrix;
e) have an ability to exit from mitochondrial matrix in the deprotonated form.

Preferred compound of general formula (I) is the following group of compounds of structure (II): 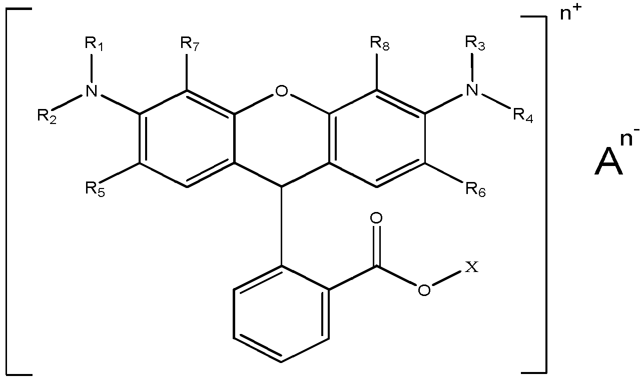 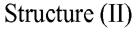 and/or reduced form thereof,
where:
R1 and R3 - hydrogen atoms, R2 and R4 independently of one another - hydrogen atoms or substituted or unsubstituted C1-C4 alkyl groups; R2, linked to R5; or R4 to R6, form thus substituted or unsubstituted 1,3-propylene;
R5, R6, R7, and R8 - independently of one another are hydrogen atom or methyl;
«A» - pharmacologically acceptable anion;
«n» - integer from 1 to 3.

The most preferred compounds of general formula (I) are compounds of formula (1) - C10R19 and (2) - C12R19: 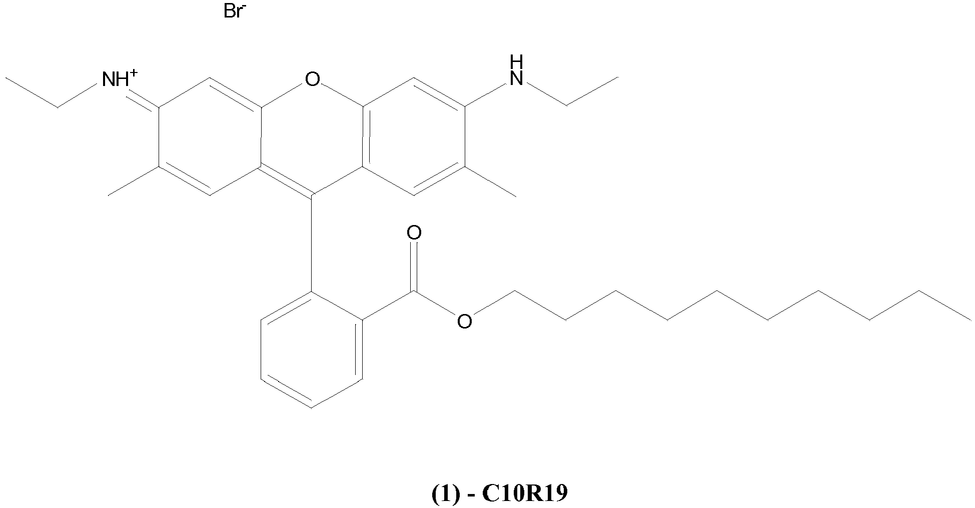 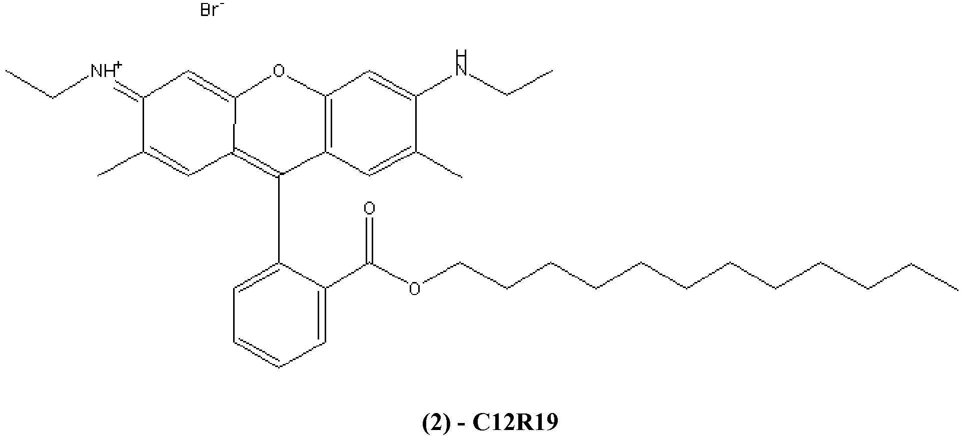 and/or reduced forms thereof, with proviso that, along with Br⁻, any other pharmacologically acceptable anion, such as Cl⁻, SO₄²⁻, etc., can be selected as an anion;

A key aspect of the present invention is pharmaceutical composition comprising effective amount of mitochondria-targeted protonophores (same as compounds of structure (I)) intended to prevent or treat certain diseases in humans and animals, at which mild uncoupling of mitochondria is effective. It should be noted that medical purpose of the composition (indications for use) is the most crucial aspect of the composition, largely defining its content, dosage and mode of application etc.

A further aspect of the present invention is use of mitochondria-targeted protonophores for manufacturing of a pharmaceutical composition intended to prevent or treat diseases, at which stimulation of cellular metabolism, including lipid metabolism, is effective.

A further aspect of the present invention is use of mitochondria-targeted protonophores for manufacturing of medical preparations intended to prevent or treat cardiovascular pathologies including atherosclerosis, cardiac arrhythmias, ischemic heart disease, myocardial infarction, renal ischemia or infarction, brain stroke, and various diseases and pathological conditions resulting from disorders of blood circulation or oxygen supply to tissues and organs.

Aspects of the present invention are also:
- application of mitochondria-targeted protonophores for treatment of pathological conditions that are consequences of non-ischemic circulatory disorders including consequences of hemorrhagic hypovolemia caused by destruction of vessels or violation of their permeability, and also hypovolemia caused by vasodilators;
- application of mitochondria-targeted protonophores for treatment of pathological conditions that are consequences of non-ischemic circulatory disorders including hypovolemia caused by loss of water through skin, lungs, gastrointestinal tract or kidneys, including hypovolemia provoked by diuretics;
- application of mitochondria-targeted protonophores for treatment of consequences of hypoxemic hypoxia caused by lack of oxygen in arterial blood, including hypoxia caused by lack of oxygen in inhaled air;
- application of mitochondria-targeted protonophores for treatment of consequences of anemic hypoxia caused by decrease in blood oxygen capacity by virtue of the fall of the amount of hemoglobin in blood or a change in hemoglobin's state;
- application of mitochondria-targeted protonophores for prevention of development or treatment of unwanted changes in cells, tissues or organs, natural blood flow or oxygen supply to which is limited or terminated during medical intervention including during apheresis, reducing or eliminating blood flow to cells, tissues or organs aimed at their further conservation and/or transplantation, as well as various surgical procedures;
- application of mitochondria-targeted protonophores for increase in lifespan of a human or an animal, as well as at disease associated with aging and increased oxidative stress;
- application of mitochondria-targeted protonophores to combat eye diseases associated with oxidative stress and/or massive loss of retinal cells or other types of cells involved in processes for ensuring vision;
- application of mitochondria-targeted protonophores for treatment or prevention of diseases associated with massive programmed cell death in tissues and organs and/or associated with distribution of signals in affected tissue which trigger programmed cell death;
- application of mitochondria-targeted protonophores for prevention and/or treatment of cardiovascular diseases for which the key role of programmed cell death, apoptosis or necrosis was shown;
- application of mitochondria-targeted protonophores in transplantation to combat tissue rejection and preserve graft material;
   application of mitochondria-targeted protonophores in cosmetology to overcome effects of burns, improve healing of wounds and surgical sutures;
   application of mitochondria-targeted protonophores in biotechnology to increase viability of animal cell culture or human cell culture for research and technological needs;
   application of mitochondria-targeted protonophores to increase productivity of animal, human, plant or fungal cell culture, when used for production of pharmaceutical preparations, proteins, antibodies;
   application of mitochondria-targeted protonophores to increase productivity of whole plants, when used for production of pharmaceutical preparations: proteins, antibodies;
- application of mitochondria-targeted protonophores for treatment and prevention of mental and psychological disorders including clinical depression and psychosis;
- application of mitochondria-targeted protonophores for treatment of parasitic diseases in humans and animals caused by fungi, Trypanosoma, Leishmania, helminths and other parasites;
- application of mitochondria-targeted protonophores for suppression of spermatogenesis and use as male contraceptive agents;

Diseases associated with disorders of cell metabolism refer to: insulin-dependent diabetes mellitus, insulin-dependent diabetes mellitus with coma, insulin-dependent diabetes mellitus with ketoacidosis, insulin-dependent diabetes mellitus with renal disease, insulin-dependent diabetes mellitus with eye damage, insulin-dependent diabetes mellitus with neurological complications, insulin-dependent diabetes mellitus with peripheral circulatory disorders, insulin-dependent diabetes mellitus with multiple complications, insulin-dependent diabetes mellitus without complications, non-insulin-dependent diabetes mellitus, non-insulin-dependent diabetes mellitus with coma, non-insulin-dependent diabetes mellitus with ketoacidosis, non-insulin-dependent diabetes mellitus with renal disease, non-insulin-dependent diabetes mellitus with eye damage, non-insulin-dependent diabetes mellitus with neurological complications, non-insulin-dependent diabetes mellitus with peripheral circulatory disorders, malnutrition-related diabetes mellitus, localized fat deposition, obesity, obesity caused by excessive energy intake, obesity caused by taking drugs, extreme obesity accompanied by alveolar hypoventilation, hypervitaminosis A, hypercarotinemia, vitamin B6 overdose syndrome, hypervitaminosis D, effects of excess food supply, disorders of aromatic amino acid metabolism, classic phenylketonuria, other types of hyperphenylalaninemia, disorders of tyrosine metabolism, albinism, other disorders of aromatic amino acid metabolism, disorders of branched-chain amino acid metabolism and fatty acid metabolism, maple syrup urine disease, violation of transport of amino acids, disorders of sulfur-containing amino acid metabolism, urea cycle disorders, disorders of lysine and hydroxylysine metabolism, disorders of ornithine metabolism, disorders of glycine metabolism, lactose intolerance, inborn lactose deficiency, secondary lactase deficiency, glycogen storage diseases, disorders of fructose metabolism, disorders of galactose metabolism, disorders of pyruvate metabolism and gluconeogenesis, disorders of sphingolipid metabolism and other lipid storage diseases, GM2-gangliosidosis, sphingolipidosis, neuronal ceroid lipofuscinoses, disorders of glycosaminoglycan metabolism, mucopolysaccharidosis type I, mucopolysaccharidosis type II, disorders of glycoprotein metabolism, defects in post-translational modification, disorders of glycoprotein degradation, pure hypercholesterolemia, pure hyperglyceridemia, mixed hyperlipidemia, hyperchylomicronaemia, lipoprotein deficiency, disorders of purine and pyrimidine metabolism, hyperuricemia without signs of inflammatory and gouty arthritis, Lesch-Nyhan syndrome, disorders of porphyrin and bilirubin metabolism, hereditary erythropoietic porphyria, porphyria cutanea tarda, defects of catalase and peroxidase, Gilbert's syndrome, Crigler-Najjar Syndrome, disorders of mineral metabolism, cystic fibrosis, amyloidosis, hyperosmolarity and hypernatremia, hypoosmolarity and hyponatremia, acidosis, alkalosis, nutrition disorders and metabolic disorders in other diseases.

Ischemic heart disease refers to: angina [angina pectoris], unstable angina pectoris, unstable angina pectoris with hypertension, angina pectoris with documented spasm, angina pectoris with documented spasm with hypertension, acute myocardial infarction, acute transmural anterior wall myocardial infarction, acute transmural anterior wall myocardial infarction with hypertension, acute transmural inferior wall myocardial infarction, acute transmural inferior wall myocardial infarction with hypertension, acute subendocardial myocardial infarction, acute subendocardial myocardial infarction with hypertension, recurrent myocardial infarction, recurrent inferior wall myocardial infarction, some current complications following acute myocardial infarction, hemopericardium as current complication following acute myocardial infarction, atrial septal defect as current complication following acute myocardial infarction, ventricular septal defect as current complication following acute myocardial infarction, thrombosis of atrium, auricular appendage, and ventricle as current complications following acute myocardial infarction, other forms of acute ischemic heart disease, coronary thrombosis that does not lead to myocardial infarction, Dressler's syndrome, Dressler's syndrome with hypertension, chronic ischemic heart disease, atherosclerotic cardiovascular disease, old myocardial infarction, cardiac aneurysm, cardiac aneurysm with hypertension, coronary artery aneurysm, coronary artery aneurysm with hypertension, ischemic cardiomyopathy, asymptomatic myocardial ischemia;

Other heart diseases refer to: acute pericarditis, acute nonspecific idiopathic pericarditis, infectious pericarditis, chronic adhesive pericarditis, chronic constrictive pericarditis, pericardial effusion (noninflammatory), acute and subacute endocarditis, acute and subacute infective endocarditis, non-rheumatic mitral valve lesions, mitral valve insufficiency, mitral valve prolapse, non-rheumatic mitral valve stenosis, non-rheumatic aortic valve lesions, aortic valve stenosis, aortic valve insufficiency, non-rheumatic tricuspid valve lesions, non-rheumatic tricuspid valve stenosis, non-rheumatic tricuspid (valve) insufficiency, pulmonary valve lesions, pulmonary valve stenosis, pulmonary valve insufficiency, pulmonary valve stenosis with insufficiency, acute myocarditis, infectious myocarditis, isolated myocarditis, cardiomyopathy, dilated cardiomyopathy, obstructive hypertrophic cardiomyopathy, eosinophilic endomyocardial disease, endocardial fibroelastosis, alcoholic cardiomyopathy, cardiomyopathy caused by influence of drugs and other external factors, atrioventricular block and left bundle branch (the His bundle) block, first-degree atrioventricular block, second-degree atrioventricular block, third-degree atrioventricular block (complete heart block), pre-excitation syndrome, cardiac arrest, cardiac arrest with successful restoration of cardiac activity, paroxysmal tachycardia, recurrent ventricular arrhythmia, supraventricular tachycardia, ventricular tachycardia, atrial fibrillation and atrial flutter, ventricular fibrillation and ventricular flutter, premature atrial depolarization, premature ventricular depolarization, sick sinus syndrome, heart failure, congestive heart failure, left ventricular failure, myocardial degeneration.

Stroke refers to both hemorrhagic and ischemic brain strokes, and their symptoms.

Renal ischemia or infarction refer to: periarteritis nodosa, Wegener's granulomatosis, hemolytic-uremic syndrome, idiopathic thrombocytopenic purpura, syndrome of disseminated intravascular coagulation (DIC), renal artery ischemia or renal artery infarction (extrarenal part), renal atherosclerosis, congenital renal stenosis, Goldblatt kidney, acute nephritic syndrome, minor glomerular disorders, focal and segmental glomerular lesions, diffuse membranous glomerulonephritis, diffuse mesangial proliferative glomerulonephritis, diffuse endocapillary proliferative glomerulonephritis, diffuse mesangiocapillary glomerulonephritis, dense deposit disease, diffuse crescentic glomerulonephritis, rapidly progressive nephritic syndrome, recurrent and persistent hematuria, chronic nephritic syndrome, nephrotic syndrome, nephritic syndrome.

Different eye pathologies refer to different forms of macular degeneration (MD) and other related symptoms such as: atrophic (dry) MD, exudative (wet) MD, age-related maculopathy (ARM), choroidal neovascularization, detached pigment retinal epithelium (PED), atrophy of pigment retinal epithelium (RPE). The term "macular degeneration (MD)" also comprises all eye diseases irrelevant to age-related changes in a human organism such as vitelliform degeneration of Best, Stargardt disease, juvenile macular dystrophy, Behr's disease, Sorsby's dystrophy, Doyne honeycomb retinal dystrophy. Symptoms related to macular degeneration refer to: drusen surrounded by white-yellow spots, submacular discoid scar of tissues, choroidal neovascularization, detached pigment retinal epithelium (PED), atrophy of pigment retinal epithelium (RPE), anomalous expansion of choroidal blood vessels, blurred or disturbed vision area, central dead point, pigment anomalies, mixed layer of thin granulation located on the inner side of Bruch's membrane, thickening and lowered permeability of Bruch's membrane.

The causes of macular degeneration include, but are not limited to: genetic or physical trauma, diseases such as diabetes, or infections, in particular, bacterial.

Wounds and other surface injuries refer to wounds associated with damage of skin epithelium, corneal epithelium, surfaces of the gastrointestinal tract, pulmonary epithelium and inner surfaces of liver vessels, blood vessels, uterus, vagina, urethra, or respiratory tract and also wounds and other surface damage associated with prolonged epithelial defects and recurrent epithelial erosions, such as: surgical wounds, excision wounds, blisters, ulcers, other lesions, abrasions, erosions, lacerations, cuts, suppurating wounds, boils and thermal or chemical bums. Wounds can be caused by mechanical damage, and result from other diseases, such as: diabetes, corneal dystrophy, uremia, malnutrition, vitamin deficiency, obesity, infection, immunodeficiency, or complications associated with systematic use of steroids, radiation therapy, non-steroidal anti-inflammatory drugs and anticancer drugs.

Mental and psychological disorders refer to (and described in detail in the International Classification of Diseases, 10th Revision) clinically defined group of symptoms or behavioral traits that in most cases cause distress and interfere with personal functioning, such as clinical depression, schizophrenia, schizotypal and delusional disorders, neurotic and behavioral symptoms, personality disorders and emotional disorders.

Parasitic diseases refer to infectious diseases caused by protozoa (amoebas, Leishmania, Lamblia, Plasmodium, trypanosomes, balantidiums, Pneumocystis, Toxoplasma, etc.), parasitic worms (helminths), arthropods (insects and mites), pathogenic microorganisms (bacteria, spirochetes, rickettsia, pathogenic fungi and viruses).

Application of pharmaceutical compositions related to the present invention can be both somatic and local. Procedures of administration comprise enteral, such as oral, sublingual and rectal; local, such as transdermal, intradermal and oculodermal; and parenteral. Suitable parenteral procedures of administration comprise injections, for example, intravenous, intramuscular, subdermal, intraperitoneal, intra-arterial, and other injections, and non-injecting practices, such as intravaginal or nasal, as well as procedure of administration of a pharmaceutical composition as angioplastic stent coating. Preferably, compounds and pharmaceutical compositions related to the present invention, are for intraperitoneal, intravenous, intra-arterial, parenteral or oral administration. In particular, administration can be given in form of injections or tablets, granules, capsules or other pressed or compressed form.

When a compound of structure (I) is administered as a pharmaceutical composition, a compound of structure (I) should be mixed according to formula with a suitable amount of pharmaceutically acceptable solvent or carrier so that to have the appropriate form for administration to a patient. The term "solvent" relates to diluent, auxiliary medicinal substance, filler or carrier which is mixed with a compound of structure (I) for administration to a patient. Liquors like water, and oils including petrolic, animal, vegetative and synthetic, such as peanut oil, soybean oil, mineral oil and other similar oils can be used as said pharmaceutical carriers. Normal saline solution, acacia pitch, gelatin, starch, talc, keratin, colloid silver, urea etc can serve as said pharmaceutical solvents.

Said composition can also comprise auxiliary substances, stabilizers, thickeners, lubricant and coloring agents.

Compounds and compositions related to the present invention can be administered in form of capsules, tablets, pills, pillets, granules, syrups, elixirs, solutions, suspensions, emulsions, suppositories or retarded release substances, or in any other form suitable for administration to a patient. One aspect of the present invention is application of compounds of structure (I) and compositions in form of solutions for oral and intraperitoneal, intra-arterial and intravenous administration.

Therapeutically effective amount of a compound of structure (I) required for treatment of a specific disease or symptom, depends on the nature of disease or symptom and a procedure of administration and should be determined at consultation with a physician in charge. Acceptable doses for oral administration are from 0.025 to 120000 microgram per kg of patient body weight, 25 microgram per kg of patient body weight is more preferable, and 50 microgram per kg of patient body weight is the most preferable. Acceptable doses for intravenous administration are from 0.1 to 10000 microgram per kg of patient body weight, 25 microgram per kg of patient body weight is more preferable, and 125 microgram per kg of patient body weight is the most preferable.

For optimal protection of cells, tissues and organs, compound of structure (I) as a pharmaceutical composition is administered 6-48 hours (optimally 24 hours) before ischemic exposure.

Examples of acceptable pharmaceutical compositions for oral administration:
Pharmaceutical composition - 1 - gelatin capsules:

| Ingredient | Amount (mg/capsule) |
|---|---|
| Compound of structure (I) | 0.0015-1000 |
| Starch | 0-650 |
| Starch powder | 0-650 |
| Liquid silicone | 0-15 |

Pharmaceutical composition - 2 - tablets:

| Ingredient | Amount (mg/capsule) |
|---|---|
| Compound of structure (I) | 0.0015-1000 |
| Microcrystalline cellulose | 200-650 |
| Silicon dioxide powder | 10-650 |
| Stearic acid | 5-15 |

Pharmaceutical composition - 3 - tablets:

| Ingredient | Amount (mg/capsule) |
|---|---|
| Compound of structure (I) | 0.0015-1000 |
| Starch | 45 |
| Microcrystalline cellulose | 35 |
| Polyvinylpyrrolidone (10% aqueous solution) | 4 |
| Carboxymethylcellulose, sodium salt | 4.5 |
| Talc | 1 |
| Magnesium stearate | 0.5 |

Pharmaceutical composition - 4 - suspensions:

| Ingredient | Amount (mg/5 ml) |
|---|---|
| Compound of structure (I) | 0.0015-1000 |
| Syrup | 1.25 |
| Benzoic acid solution | 0.10 |
| Carboxymethylcellulose, sodium salt | 50 |
| Flavoring | By necessity |
| Coloring | By necessity |
| Distilled water | Up to 5 ml |

An example of acceptable pharmaceutical composition in the form of solution for intraperitoneal, intra-arterial and intravenous administration (pH 6.5):

| Ingredient | Amount |
|---|---|
| Compound of structure (I) | 5 mg |
| Isotonic solution | 1000 ml |

An example of acceptable pharmaceutical composition for administration in the form of aerosol:

| Ingredient | Amount (weight percent) |
|---|---|
| Compound of structure (I) | 0.0025 |
| Ethanol | 25.75 |
| Difluorochloromethane | 70 |

An example of acceptable pharmaceutical composition for administration in the form of suppositories:

| Ingredient | Amount (mg/suppository) |
|---|---|
| Compound of structure (I) | 1 |
| Glycerides of saturated fatty acids | 2000 |

### Brief description of figures

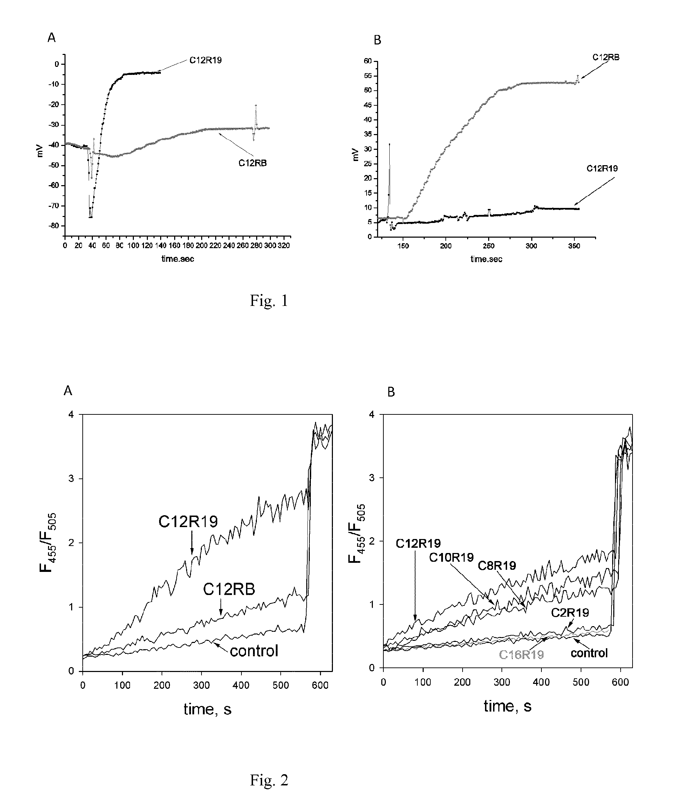

**Fig. 1****. A.** C12R19, but not C12RB, induces formation of proton diffusion potential across planar lipid bilayer membrane prepared from DPhPC. Concentrations of C12R19 and C12RB are 1 µM. pH_cis = 7, pH_trans = 8. **B.** Formation of potential upon creation of gradient of penetrating cations C12RB and C12R19 (1 µM on cis side) at the same pH on both sides (pH = 7).
**Fig. 2****. A.** Dissipation of pH gradient in membranes of pyranine-loaded liposomes induced by C12R19 and C12RB. Intraliposomal pH was estimated based on the ratio of the intensities of fluorescence at 455 nm and 505 nm (excitation at 410 nm). 1 µM nigericin was added at 550 s for final pH adjustment. Control curve contained C12R19 but no valinomycin. Concentrations: C12R19 (150 nM), C12RB (150 nM), valinomycin (10 nM), lipid (75 µM). **B.** Dependence of the effect on the length of the alkyl tail. Control curve corresponds to addition of 10 nM valinomycin without protonophores. Buffer containing 10 mM MES, 10 mM MOPS, 10 mM TRICINE, and 0.1 M KCl (pH 8) was used.

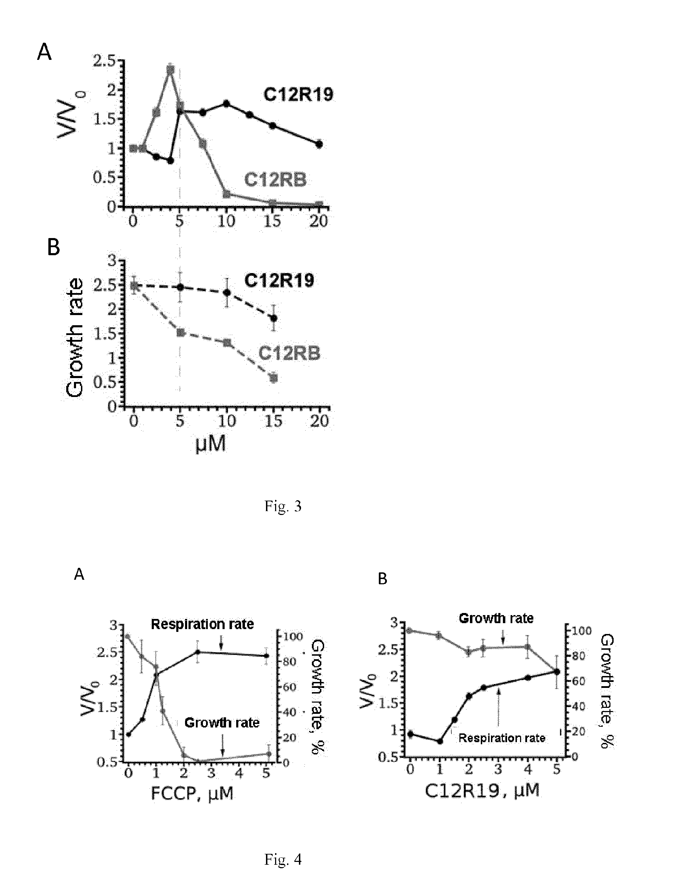

**Fig. 3****.** Comparison of properties of C12R19 and C12RB based on relative rate of oxygen consumption by whole yeast (*Saccharomyces cerevisiae*) cells **(A)** and yeast growth rate **(B).** V/V₀ corresponds to ratio of oxygen consumption rates after and before addition of uncoupler. Growth rate was defined as the number of CFU (colony-forming units) in cell suspension culture incubated for 2 hours in the presence of indicated concentration of rhodamine derivative.
**Fig. 4****.** Comparison of stimulation of respiration and suppression of growth: **(A)** cationic (C12R19), and **(B)** anionic (FCCP) uncouplers in yeast (*Saccharomyces cerevisiae*) cells grown on nonfermentable substrate (glycerol). Stimulation of respiration was estimated based on the ratio of oxygen consumption rate in the presence of uncoupler (V) to initial rate (V₀). Growth rate was assessed by increase in the number of cells for a given time interval. Growth rate in the absence of uncoupler was taken as 100%.

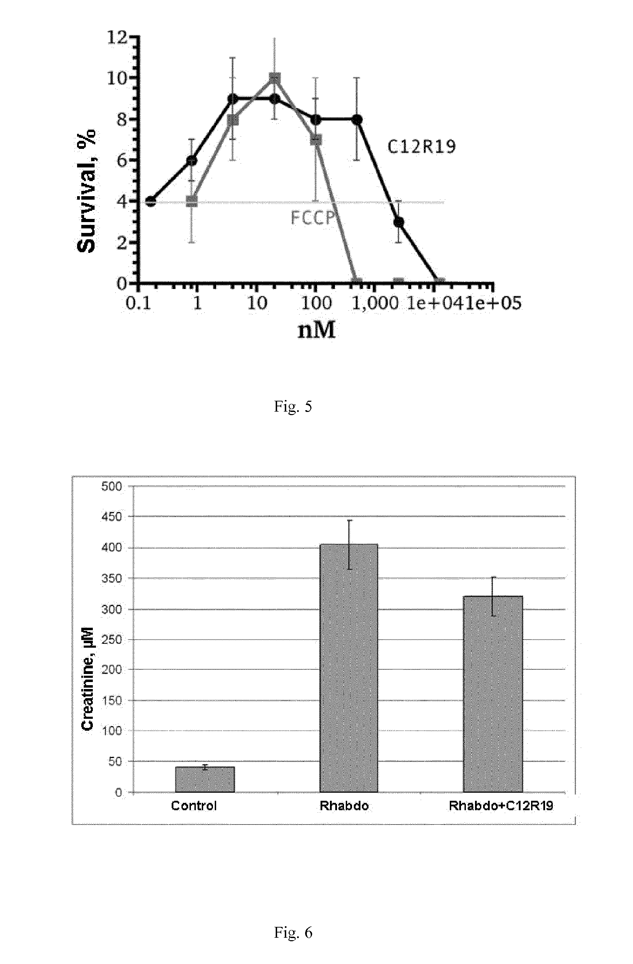

**Fig. 5****.** Comparison of resistance of yeast cells to 80 µM amiodarone in the presence of different concentrations of anionic (FCCP) and cationic (C12R19) uncouplers.
**Fig. 6****.** Creatinine level in blood of rats with induced rhabdomyolysis. Histogram bar "control" indicates level of creatinine in control group of animals; "rhabdo" - creatinine level in animals with rhabdomyolysis; "rhabdo+C12-R19" - creatinine level in animals with rhabdomyolysis and treatment with C12R19. All data are presented as mean ± standard error of measurement.

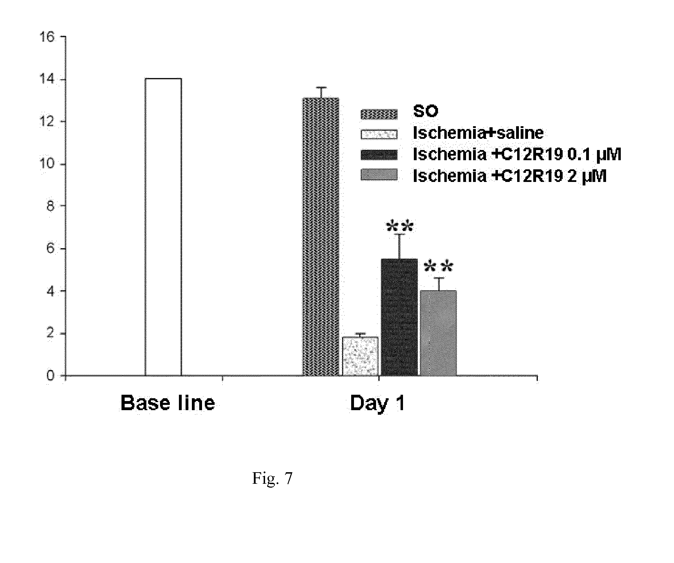

**Fig. 7****.** Results of behavioral tests in rats before and at the first day after induction of ischemia. Bar "baseline" indicates final points in animals before surgery; "SO" - points in sham-operated animals; "ischemia+saline" - points in ischemized animals without treatment, with saline solution instead of the preparation; "ischemia +C12R19 0.1 µM" and "ischemia +C12R19 2 µM" - points in ischemized animals with treatment with preparation C12R19 at indicated concentrations. Statistically significant (p<0.01) reduction in severity of neurological deficit in rats treated with C12R19, denoted as "**".

The following non-limiting Examples illustrate the preparation and use of compounds of structure I but should not be understood as limiting the invention as modifications in materials and methods will be apparent to the skilled person. The following examples should not be construed as limiting the scope of this disclosure.

### Experimental examples

### 1) Synthesis of compounds of structure (I) C12R19 and C10R19.

Dodecyl ether rhodamine 19 **(C12R19, 1)** and decyl ether rhodamine 19 **(C10R19, 2)** were prepared by acylation of dodecyl bromide (3) or decyl bromide (4), respectively, previously obtained from rhodamine 19 (5) its cesium salt (6). **(Scheme 1).**

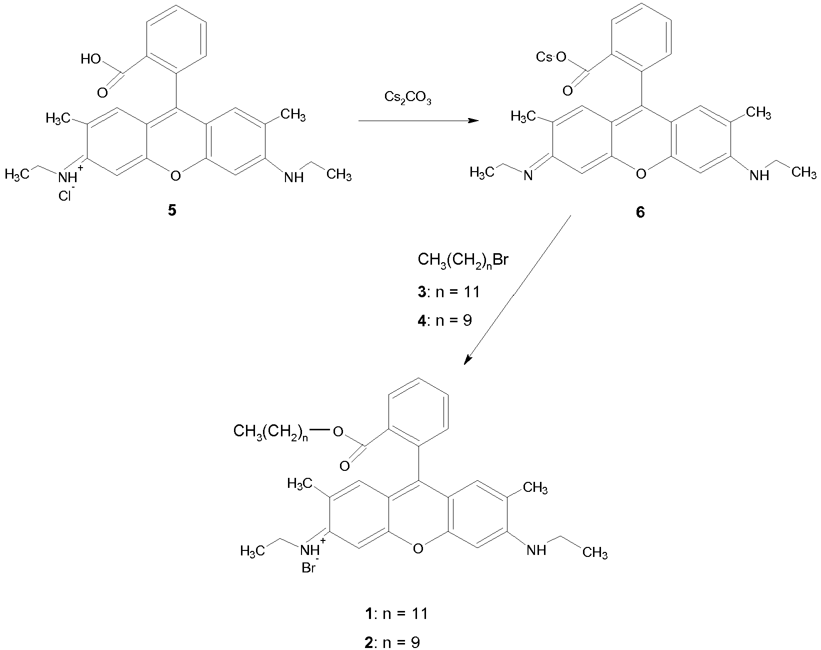

### Methodology

### Decyl-2-[(3E)-6-(ethylamino)-3-(ethylimino)-2,7-dimethyl-3H-xanthen-9-yl]benzoate bromide (C10R19).

### OR

### Decyl ether rhodamine 19 bromide (C10R19).

*Cs-salt of rhodamine 19.* 1 ml of 2 N aqueous solution of cesium carbonate was added to solution of rhodamine 19 (240 mg, 0.53 mmol) in 6 mL of methanol, the solution was heated to boiling, and then cooled to 4 °C and kept at that temperature for 12 hours. The precipitate was filtered, washed with cold methanol and dried under vacuum at 60 °C. Cesium salt of rhodamine 19 in the form of dark purple solid substance (238 mg, 0.435 mmol, 82% yield) was obtained.

Mixture of Cs-salt of rhodamine 19 (137 mg, 0.25 mmol) and 1-bromodecane (78 µl, 83 mg, 0.375 mmol) in 1 mL of DMF was kept in a sealed tube at 60 °C for 72 h. The solvent was removed under vacuum and the residue was purified by column chromatography on silica gel column with chloroform:methanol (4:1, v/v). The product in the form of dark red solid substance (135 mg, 0.243 mmol, 97.2%) was obtained. TLC: *R_{f}* (CH₃Cl - iProOH - CH₃OH - AcOH - H₂O 80:15:8:3:1) 0.25; *R_{f}* (CH₂Cl₂ - CH₃OH, 9:1) 0.32; HPLC: t_{R} = 5.195 min (70-95 % B for 11 min; A: 0.05% aqueous TFA; B: 0.05% TFA in MeCN; ESI MS: m/z (MH+) calculated for C₃₆H₄₆N₂O₃: 554.35, found: 555.39; UV (C₂H₅OH): λₘₐₓ 248, 348, 528 nm (ε = 76000 cm⁻¹*M⁻¹ ); fluorescence (CH₃OH): λ_{EX} 528 nm, λ_{EM} 547 nm.

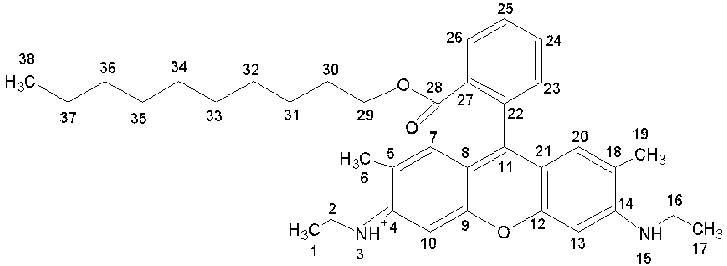

NMR ¹H (400.13 MHz, CDCl₃, ppm): 0.9 (t, 3H, 38 - CH₃); 1.3 (m, 22H, 1.17, 30-37 - 2CH₃, 8CH₂); 2.37 (s, 6H, 6 and 19 - 2CH₃); 3.64 (m, 4H, 2 and 16 - 2CH₂); 4.0 (t, 2H, 29 - CH₂); 6.69 (s, 2H, 7 and 20 - 2CH); 6.79 (s, 2H, 13 and 10 - 2CH); 7.45 (m, 2H, 3 and 15 - 2NH); 7.8 (m, 2H, 24 and 25 - 2CH); 8.37 (d, 1H, 26 - CH).

NMR ¹³C (100.61 MHz, CDCl₃, ppm): 13.94 (1 and 17 - 2CH₃),14.16 (38 - CH₃); 18.89 (6 and 19 - 2CH₃); 22-32 (30-37 - 8CH₂); 38.44 (2 and 16 - 2CH₂); 65.82 (29 - CH₂); 93.68 (7 and 20 - 2CH); 113.39 (C₈ and C₂₁); 126.29 (C₅ and C₁₈); 128.43 (10 and 13 - 2CH); 130.07 and 130.14 (23 and 24 - 2CH); 130.30 (C₁₁); 131.37 (26 - CH); 132.78 (25 - CH); 133.99 - (C₂₇); 155.96 (C₄ and C₁₄); 156.80 (C₂₂); 157.10 ( C₉ and C₁₂); 165.26 (28 - CO).

### Dodecyl-2-[(3E)-6-(ethylamino)-3-(ethylimino)-2,7-dimethyl-3H-xanthen-9-yl]benzoate bromide (C12R19)

### OR

**Dodecyl ether rhodamine 19 bromide (C12R19)** was obtained according to the method described for **C10R19,** based on cesium salt of rhodamine 19 (70 mg, 0.128 mmol) and 1-bromododecane (46 µl, 48 mg, 0.192 mmol) in 1.5 mL of DMF. The product was isolated by column chromatography on silica gel column with chloroform:methanol (9:1, v/v), dark red solid substance was obtained (80 mg, 0.12 mmol, 94%). TLC: *R_{f}* (CH₃Cl - iPrOH - CH₃OH - AcOH - H₂O, 80:15:8:3:1) 0.36; *R_{f}*(CH₂Cl₂ - CH₃OH, 9:1) 0.37; HPLC: t_{R} = 7.159 min (70-95 % B for 11 min; A: 0.05% aqueous TFA; B: 0.05% TFA in MeCN; ESI MS: m/z (MH+) calculated for C₃₈H₅₀N₂O₃: 582.38, found: 583.59; UV (C₂H₅OH): λₘₐₓ 248, 348, 528 nm (ε = 76000 cm⁻¹*M⁻¹); fluorescence (CH₃OH): λ_{EX} 528 nm, λ_{EM} 547 nm.

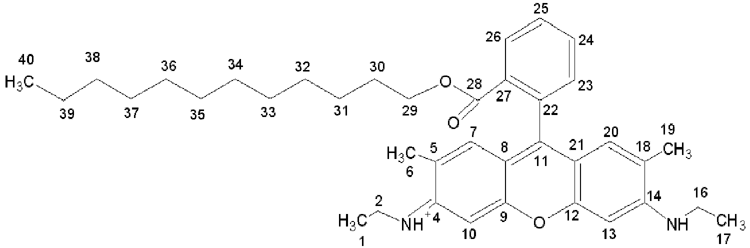

NMR ¹H (400.13 MHz, CDCl₃, ppm): 0.85 (t, 3H, 40 - CH₃); 1.15 (m, 20H, 30-39 - 10CH₂); 1.38 (t, 6H, 1 and 17 - 2CH₃); 2.27 (s, 6H, 6 and 19 - 2CH₃); 3.56 (m, 4H, 2 and 16 - 2CH₂); 3.94 (t, 2H, 29 - CH₂); 6.66 (s, 2H, 7 and 20 - 2CH); 6.75 (m, 4H, 10, 13 and 3 and 15 - 2CH and 2NH); 7.25 (d, 1H - 23 CH); 7.77 (m, 2H - 24 and 25 - 2CH); 8.22 (d, 1H, 26 - CH).

NMR ¹³C (100.61 MHz, CDCl₃, ppm): 14.04 (1 and 17 - 2CH₃), 14.13 (40 - CH₃); 19.04 (6 and 19 - 2CH₃); 22-32 (30-39 - 10CH₂); 38.48 (2 and 16 - 2CH₂); 65.84 (29 - CH₂); 93.92 (7 and 20 - 2CH); 113.61 (C₈ and C₂₁); 126.12 (C₅ and C₁₈); 128.61 (10 and 13 - 2CH); 130.13 and 130.17 (23 and 24 - 2CH); 130.17 (C₁₁); 131.37 (26 - CH); 132.83 (25 - CH); 133.86 - (C₂₇); 155.85 (C₄ and C₁₄); 157.43 (C₂₂); 157.2 (C₉ and C₁₂); 165.20 (28 - CO).

### 2) Study of protonophore activity of cationic uncouplers on "black membrane" model.

To test penetrating ability of mitochondria-targeted compounds of structure (I), a method based on the ability of ions to penetrate through the bilayer phospholipid membrane moving along concentration gradient was used. The two chambers filled with an aqueous solution are separated by the bilayer membrane, the compound tested is added to one of the chambers. If a charged compound is capable of penetrating through the bilayer membrane, fast diffusion of the compound from the chamber with its high concentration to the chamber with its low concentration occurs, thus difference in the potentials is formed across the membrane. For ions carrying one charge and capable of passive penetrating through the membrane, the 10-fold concentration gradient allows to produce the potential of 60 mV at room temperature and ideal permeability (according to the Nernst equation).

This method was used in various studies on the ability of ions to penetrate through a bilipid membrane and is described in detail in article: Starkov AA, Bloch DA, Chernyak BV, Dedukhova VI, Mansurova SA, Symonyan RA, Vygodina TV, Skulachev VP, 1997, Biochem. Biophys Acta, 1318, 159-172. With this method, C12-R19 (compound of structure (I)) was tested.

**Figure 1** shows results of measurement of diffusion potential (ΔΨ) on lipid bilayer formed from lipid diphytanoyl phosphatidylcholine at different pH values on both sides of the membrane. It is known that addition of protonophore to such a system should lead to formation of the electric potential due to the flow of protons. **Figure 1A** shows that C12R19, but not C12RB, can generate potential in such a system. In addition, experiments were conducted on potential generation upon creation of gradients of penetrating cations themselves, and it was shown that C12RB induced the potential upon addition at one side **(****Figure 1B****),** whereas C12R19 did not. This can be explained by shunting of potential due to induction of proton conductivity in the presence of C12R19.

### 3) Dissipation of pH gradient in membranes of pyranine-loaded liposomes induced by cationic uncouplers.

**Figure 2A** shows kinetics of dissipation of pH gradient in membranes of liposomes induced by 0.15 µM C12R19 or C12RB. This method was used in various studies on the protonophore activity and is described in detail in article: Chen Y, Schindler M, Simon SM (1999) J Biol Chem 274:18364-18373. Addition of C12R19 led to significant changes of intraliposomal pH on the time-scale of minutes indicating a protonophore activity **(****Figure 2A****,** curve C12R19). FCCP showed significantly greater activity (data not shown). It is essential that C12RB had practically no activity in said system **(****Figure 2A****,** curve C12RB). Said measurements were performed in the presence of valinomycin, the K⁺ ionophore. In the absence of valinomycin, pH changes did not occur, as shown by the example of the effect of C12R19 in **Figure 1****,** curve "control". Perhaps this effect is associated with formation of the membrane electrical potential which prevents transport of protons without valinomycin. Control experiments showed that valinomycin itself does not induce proton flux on liposomes (data not shown).

**Figure 2B** shows the effect of alkyl derivatives of rhodamine 19 having a different tail length. There is an optimum at tail length from 10 to 12 carbon atoms.

The difference in protonophore activity between C12R19 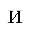 C12RB is due to the differences in the structures of said two rhodamines (rhodamines 19 and B, respectively). Rhodamine 19 cationic group contains protonable nitrogen atoms while rhodamine B has no such atoms. This apparently explains the difference in the protonophore activity.

Thus, it was shown that C12R19 has protonophore activity.

### 3) Comparison of uncoupling properties of C12R19 and C12RB based on rate of oxygen consumption by whole yeast cells.

Simulation of respiration of whole yeast cells reflects uncoupling properties of tested compound. **Figure 3A** shows that addition of C12RB to cell suspension led to significant increase in the rate of respiration at a concentration of 4 µM but further increase in concentration caused inhibition of respiration. At the same time, addition of C12R19 did not reduce the rate of respiration up to a concentration of 15 µM.

Under similar conditions experiments determining yeast growth rate in the presence of a given concentration of uncoupler were performed. **Figure 3B** shows that in the presence of 5 µM C12R19, significant reduction in the growth rate of the yeast *Saccharomyces cerevisiae* is not observed.

**Figure 4** demonstrates the advantage of C12R19 over classical anionic uncouplers (FCCP) in the system: in a wide range of concentrations C12R19 increases respiration rate without inhibiting yeast growth **(****Figure 4A****),** in contrast to FCCP **(****Figure 4B****).**

Thus, we were able to show that for C12R19 there is a certain concentration range that includes the concentration of 5 µM in which there is a marked stimulation of the rate of respiration of whole yeast cells, and, therefore, also reduction of the transmembrane potential across the inner mitochondrial membrane. According to the literature, reduction of the membrane potential correlates with a decrease in the rate of formation of reactive oxygen species by mitochondria. Thus, we conclude that micromolar concentrations of C12R19 lower the transmembrane potential but do not cause the complete suppression of oxidative phosphorylation, as deduced from the absence of reduction of cell growth rate.

### 4) Cationic uncoupler C12R19 prevents yeast cell death caused by amiodarone.

We showed (Pozniakovsky AI, Knorre DA, Markova OV, Hyman AA, Skulachev VP, Severin FF. Role of mitochondria in the pheromone- and amiodarone-induced programmed death of yeast. J Cell Biol. 2005; 68(2); 257-69*.)* that uncoupler FCCP prevents yeast cell death caused by amiodarone. Apparently this is due to reduction of mitochondrial membrane potential and, as a consequence, the rate of formation of reactive oxygen species by the respiratory chain is reduced. We hypothesized that cationic uncouplers (C12R19) will be effective in this system, and the concentration range, in which they will act, will be higher than that for anionic uncouplers. To test this, we compared the survival of yeast cells treated with 80 µM amiodarone in the presence of 1-10000 nmol/L FCCP and C12R19. **Figure 5** shows that C12R19 starts to protect cells against amiodarone in the concentration range from 1 to 1000 nmol/L. At the same time, FCCP acted only in a range of concentrations from 2.5 to 100 nmol/L. Thus, the concentration range of cationic uncoupler in this system is at least an order of magnitude higher than in the case of FCCP. It should be taken into account that the uncoupling efficiency of FCCP is much higher than that of C12R19, if it is estimated based on the rate of oxygen consumption by whole yeast (*Saccharomyces cerevisiae*) cells.

### 5) Improvement in rhabdomyolysis-induced renal function when applying C12R19.

Induction of rhabdomyolysis was performed according to standard method by injecting rat leg muscles with 50% aqueous glycerol solution (ICN, USA). At the second day after ischemia, blood samples from animals were taken. Creatinine concentration in blood was determined using a CellTac blood analyzer (Nihon Kohben). Experiments on modeling of rhabdomyolysis were performed with at least 8 animals from each group. All data are presented as mean ± standard error of measurement.

Study of dynamics of development of rhabdomyolysis-induced acute renal failure showed that at the first 2 days, there is development of severe renal failure that is manifested by increase in blood concentration of the products of nitrogen metabolism including creatinine, which reaches its peak by the second day. For the treatment, the following scheme of C12R19 administration was used: intraperitoneal injection of the preparation 1 h after induction of rhabdomyolysis and then three times every 12 hours at a dose of 100 nmol/kg per injection. Thus, an animal received a total dose of 400 nmol/kg of C12R19. **Figure 6** shows the result after the treatment with C12R19 according to said scheme: 48 h after glycerol administration there was a decrease in creatinine level in blood as compared with rhabdomyolysis without treatment.

### 6) Effect of pre-treatment with SkQR1 on size of affected area of brain and neurological deficit in ischemized rats.

Cerebral ischemia in rats was induced by insertion of nylon thread with silicone coating into the middle cerebral artery. Blockage of the blood flow was maintained for 60 min, after which the thread was removed from the vessel restoring the blood flow in the middle cerebral artery. During and after the surgery the body temperature of the animal was maintained at 37 °C. Sham-operated animals were subjected to the same procedures, except for cutting vessels and insertion of thread. The volume of cerebral infarction was determined at the first day to study neuroprotective effects of preparations. This evaluation was carried out using morphometric analysis of digital images obtained by magnetic resonance tomography (MRT). All MRT experiments were performed using a BioSpec 70/30 (Bruker, Germany) with magnetic field induction of 7 T and gradient of 105 mT/m. Behavioral tests were performed 1 day before surgery and at the first day after induction of ischemia. To assess neurological disorders caused by blockage of the middle cerebral artery, a 14-point scale was used (De Ryck M., Van Reempts J., Borgers M., Wauquier A, Janssen PA. Photochemical stroke model: flunarizine prevents sensorimotor deficits after neocortical infarcts in rats. Stroke 1989; 20; 1383-1390*.).*

The overall score is the sum of the points in 7 tests that assess response of front and hind legs to tactile and proprioceptive stimulation, the presence of certain reflexes. To assess functional impairment of the legs, the following scoring system was used: 2 points - rat completely passed the test, 1 point - rat passed the test delayed by more than 2 s and/or incompletely, 0 points - rat did not respond to stimulation of the legs.

Rats were injected intraperitoneally with C12R19 at a dose of 100 nmol/kg 24 hours before induction of ischemia.

1 day after surgery, neurological deficit was determined in rats in the test "Stimulation of legs" and magnetic resonance imaging of the brain was performed. Morphometric analysis of ischemic lesions by T2-weighted magnetic resonance images showed that C12R19 administration caused reduction in the volume of ischemic damage to 80.4 ± 11.5% as compared to animals treated with saline, 100 ± 8.6%. Also, statistically significant reduction in severity of neurological deficit occurred in rats treated with C12R19 which gained 5.3 ± 1.1 points as compared to the control rats with 2.0 ± 0.2 points (p<0.01) **(****Figure 7****).**

## Claims

1. Composition comprising therapeutically effective amount of a compound of formula (I) for the increase of proton conductivity of biological membranes: 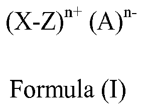 and/or reduced form thereof,
where
«X» - group comprising substituted or unsubstituted hydrocarbon chain with a length of up to 20 carbon atoms, optionally containing double or triple bonds, heteroatoms such as -S-, -N-, - O-, or optionally containing -C(O)O-, -C(O)- and/or -C(O)N(H)- groups;
«Z» - group that can be protonated under physiological pH values and able to provide targeted delivery of the whole compound into mitochondria.
«A» - counterion
«n» - integer from 1 to 3,
and/or tautomeric forms, solvates, salts, isomers or prodrug forms thereof,
with proviso that X-Z combination should:
a) have an ability to be protonated at pH values typical to cell cytoplasm and have pK from 4 to 11;
b) have enough lipophilicity to penetrate mitochondria in the protonated form;
c) provide electrochemical potential-dependent penetration in mitochondria;
d) have an ability to be deprotonated under alkalescent pH conditions typical for mitochondrial matrix;
e) have an ability to exit from mitochondrial matrix in the deprotonated form.
With another proviso that the compound of formula (I) is not C12R19:

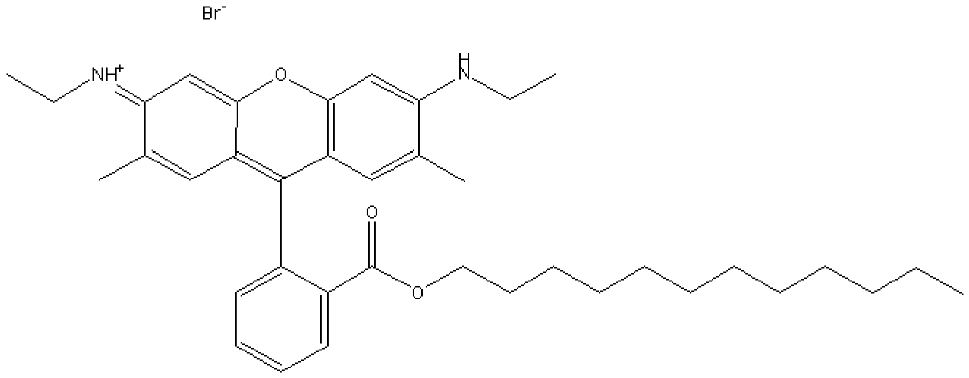

2. Composition as claimed in claim 1, wherein compound of structure (I) is compound of formula (II): 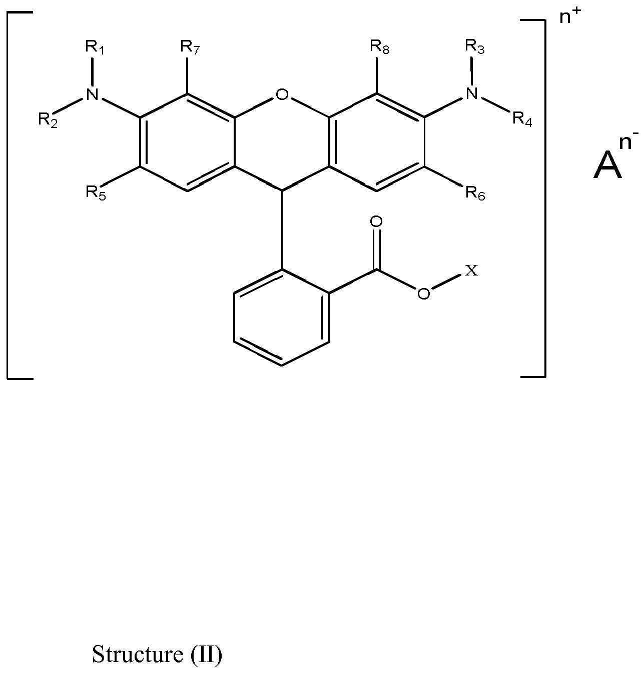 and/or reduced form thereof,
where:
R1 and R3 - hydrogen atoms, R2 and R4 independently of one another - hydrogen atoms or substituted or unsubstituted C1-C4 alkyl groups; R2, linked to R5; or R4 to R6, form thus substituted or unsubstituted 1,3-propylene;
R5, R6, R7, and R8 - independently of one another are hydrogen atom or methyl;
«A» - pharmacologically acceptable anion;
«n» - integer from 1 to 3.
With proviso that the compound of formula (I) is not C12R19:

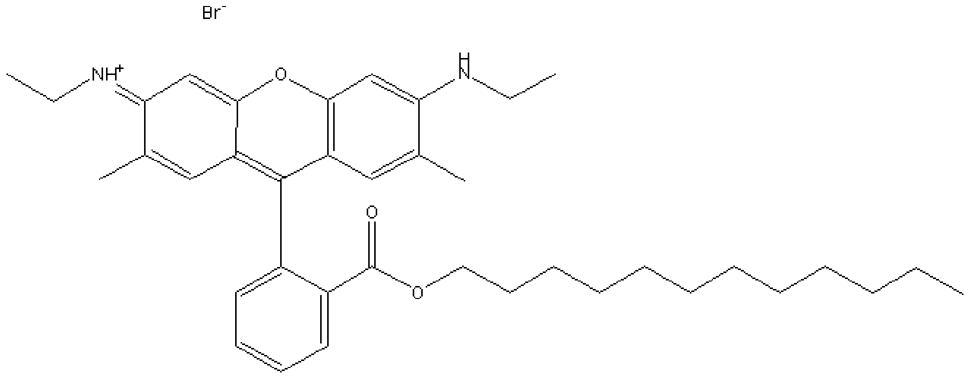

3. Composition as claimed in claim 2, wherein compound of structure (II) is compound of formula (1): 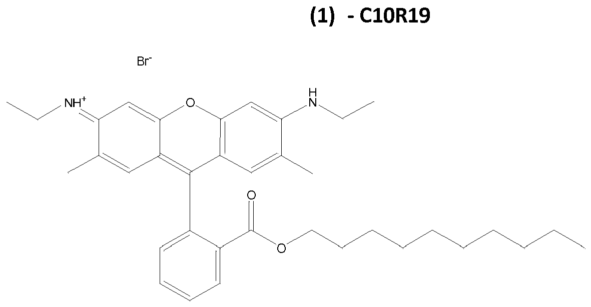 and/or reduced forms thereof, with proviso that, along with Br⁻, any other pharmacologically acceptable anion can be selected as an anion.

4. Composition comprising therapeutically effective amount of compound **C12R19:** 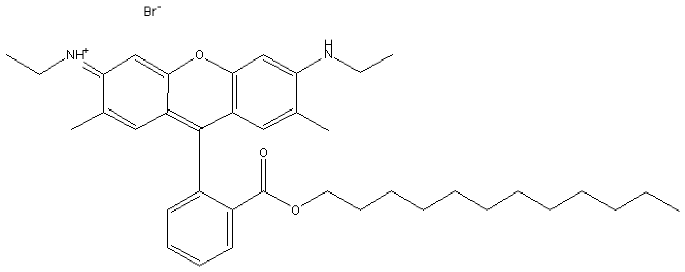 and/or reduced forms thereof, with proviso that, along with Br⁻, any other pharmacologically acceptable anion can be selected as an anion.

5. Application of compositions claimed in claims 1-4 for manufacturing of a pharmaceutical composition for reducing the amount of free radicals and reactive oxygen species in a cell.

6. Application of compounds claimed in claims 1-4 for manufacturing of a pharmaceutical composition for stimulating cell metabolism.

7. Application according to claims 5-6, when a cell, being normal or cancer, or stem cell, is in a human or other mammal; is a plant cell and/or is part of a plant at any stage of its development including genetically modified one; is in a culture of plant cells or protoplasts; is a fungal cell and/or in a culture of fungal cells; including to increase viability and/or productivity of cells producing preparations, pharmacologically applicable proteins, peptides, antibodies.

8. Application according to claim 7 for treatment of obesity including pathological forms thereof, and also for treatment of diseases associated with a metabolic disorder.

9. Application according to claim 7 for treatment of diabetes, complications caused by diabetes and also for treatment of diseases with concomitant diabetes.

10. Application according to claim 7 for treatment of a disease, at which a decrease in the amount of free radicals and reactive oxygen species in an organism is useful.

11. Application according to claim 7 for protection of healthy cells from damage during chemotherapy, radiotherapy or photodynamic therapy of cancer; during the decontamination of blood or other substance containing healthy cellular elements by means of free radicals, reactive oxygen species or substances forming them.

12. Application according to claim 7 for cosmetic procedures, healing of surgical sutures, preventing damage to a healthy tissue during surgery, healing or preventing burn tissue lesions, against inflammation, for conservation of graft material, to combat rejection of transplanted tissues and organs.

13. Application of composition according to any of claims 1-4 for increase in lifespan of an organism, the fight against aging; including application in combination with hormone therapy, particularly in combination with epiphyseal hormones, thyroid hormones including dihydroepiandrosterone or melatonin.

14. Compound of general formula (III) 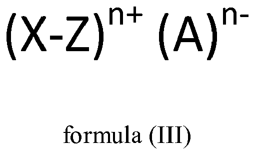 and/or reduced form thereof,
where:
«X» - group comprising substituted or unsubstantiated hydrocarbon chain with a length of up to 20 carbon atoms, optionally containing double or triple bonds, heteroatoms such as -S-, - N-, -O-, or optionally containing -C(O)O-, -C(O)- and/or -C(O)N(H)- groups;
«Z» - group that can be protonated under physiological pH values and able to provide targeted delivery of the whole compound into mitochondria.
«A» - counterion
«n» - integer from 1 to 3,
and/or tautomeric forms, solvates, salts, isomers or prodrug forms thereof,
with proviso that X-Z combination should:
a) have an ability to be protonated at pH values typical to cell cytoplasm and have pK from 4 to 11;
b) have enough lipophilicity to penetrate mitochondria in the protonated form;
c) provide electrochemical potential-dependent penetration in mitochondria;
d) have an ability to be deprotonated under alkalescent pH conditions typical for mitochondrial matrix;
e) have an ability to exit from mitochondrial matrix in the deprotonated form.
